# DEMANDE DE BREVET EUROPEEN

(11) **EP 2 361 513 A1**
(43) Date de publication de la demande: **31.08.2011**
(21) Numéro de dépôt: 11152386.6
(22) Date de dépôt: 27.01.2011
(51) Int. Cl.: A23L 1/30, A23L 1/305, A61K 35/60, A61K 31/202, A61P 3/04, A61P 25/24, A61P 37/04

(54) **Ingredient nutritionnel riche en DHA et EPA**

(30) Priorité: 27.01.2010 FR 1050547
(71) Demandeur: Polaris, 29170 Pleuven (FR)
(72) Inventeur: Breton, Gildas, 29750 Loctudy (FR); Lozachmeur, Stéphane, 29170 Benodet (FR)
(74) Mandataire: Warcoin, Jacques

(57) **Abrégé**

L'invention concerne une poudre d'oeufs de cabillaud séchés comprenant
- des acides aminés essentiels et,
- des lipides dont au moins :
a) 50% en poids sont des phospholipides,
b) 30% des acides gras totaux desdits lipides sont des acides gras oméga-3,
c) 15% des acides gras totaux desdits lipides sont du DHA,
d) 15% des acides gras totaux desdits lipides sont de l'EPA.

## Description

L'invention concerne un ingrédient nutritionnel riche en DHA et EPA majoritairement sous forme de phospholipides et riche en acides aminés dont les 8 acides aminés essentiels.

Les études conduites par les épidémiologistes, par les nutritionnistes et les biochimistes ont conduit à la conclusion qu'il existe une compétition entre les familles d'acides gras essentiels Oméga 6 et Oméga 3 et qu'un développement harmonieux de l'organisme humain nécessite que les quantités d'Oméga 6 et Oméga 3 présentes dans notre alimentation soient dans un rapport inférieur à 5 (recommandation de l'Agence Française pour la Sécurité Sanitaire des Aliments : AFSSA).

Le rapport des apports alimentaires en acides gras Oméga 6/Oméga 3 de la population française est aujourd'hui supérieur à 10.

Il est connu que la consommation d'acide gras influence les maladies chroniques :telles que obésité, diabète, cancer, arthrite, asthme et les maladies cardiovasculaires.

L'acide docosahexaénoïque (acide 4Z,7Z,10Z,13Z,16Z,19Z-docosahexaénoïque, acide C22:6 ω-3 ou plus simplement DHA) et l'acide éicosapentaénoïque (C₂₀H₃₀O₂), acide 5Z, 8Z, 11Z, 14Z, 17Z-éicosapentaénoïque, ou encore acide gras tout-*cis* C20:5 ω-3) que l'on nomme plus simplement EPA sont des acides gras poly-insaturés faisant partie de la famille des oméga 3.

On les trouve surtout dans l'huile de poisson (foie de morue, hareng, maquereau, saumon et sardine), dans l'huile de micro-algues et dans l'huile de krill.

Ces acides gras sont indispensables au bon fonctionnement de notre organisme. Ils sont synthétisés à partir de l'acide alpha linolénique (ALA), mais, du fait du taux de conversion limité, il est important de trouver du DHA et de l'EPA dans notre alimentation.
EPA :maladies inflammatoires, articulations, équilibre émotionnel...
DHA : système nerveux central, facultés visuelles, développement du cerveau pour les bébés.

### √ Protection du système cardiovasculaire

De très nombreuses études scientifiques ont permis de confirmer l'effet cardioprotecteur des acides gras oméga 3. En prévention primaire, c'est-à-dire avant l'apparition d'un trouble cardiovasculaire, une étude a montré que plus la consommation de poisson est importante, plus le risque de développer une maladie cardiovasculaire est faible. En prévention secondaire, c'est-à-dire chez des patients ayant déjà subi un infarctus du myocarde une étude montre une diminution de 20 % de la mortalité cardiaque et de 45 % de la mort subite chez ceux qui recevaient une supplémentation en oméga 3. De plus, ces acides gras ont d'autres rôles qui viennent renforcer la protection du système cardio-vasculaire : fluidisation du sang, baisse du taux de triglycérides sanguin et diminution de l'arythmie (troubles du rythme cardiaque).

### √ Action anti-inflammatoire

L'EPA a une puissante action anti-inflammatoire. En effet, il est le précurseur d'une famille de médiateurs cellulaires, les eicosanoïdes. Contrairement à ceux issus de l'acide arachidonique , un autre acide gras polyinsaturé à 20 carbones de la famille des Oméga 6, ces eicosanoides jouent un rôle physiologique bénéfique lors du processus inflammatoire ( douleurs articulaires, maladies inflammatoires...).

Afin de rétablir notre balance Oméga6/Oméga3, de nombreux produits alimentaires sont enrichis en acide gras Oméga 3 essentiel, acide alpha-linolénique, précurseur de l'EPA et du DHA, même si nous savons que le taux de conversion est extrêmement faible. Les Oméga 3, directement sous leur forme active, EPA et DHA viennent également supplémenter des produits pour des sous-populations particulièrement en besoin d'Oméga-3 comme les nourrissons et les femmes enceintes et allaitantes.

On trouve généralement les acides gras oméga-3 sous forme de triglycérides, c'est-à-dire qu'un ou plusieurs des acides gras sont liés à un squelette de glycérol et pas sous forme d'acides gras libres. Les effets bénéfiques des acides gras oméga-3, en particulier l'EPA et le DHA, requièrent de grandes quantités journalières dans l'alimentation pratiquement impossibles à atteindre en consommant du poisson.

C'est pourquoi des additifs ou ingrédients nutritionnels se sont répandus pour supplémenter notre alimentation directement dans certains aliments comme les huiles et la margarine ou par le biais de compléments alimentaires et certains aliments spécifiques comme les laits infantiles.

Le but de l'invention est de proposer un nouvel ingrédient nutritionnel riche en DHA et EPA. Il est particulièrement avantageux car il présente près de 50% des acides gras oméga-3 sous forme de phospholipides, soit sous leur forme la plus biodisponible car étant celle utilisée dans le corps humain. Il est également particulièrement avantageux car il représente une source équilibrée de DHA et d'EPA. Il est également particulièrement avantageux car il représente une source d'acides aminés essentiels

Un acide aminé essentiel est un acide aminé qui ne peut être synthétisé *de novo* par l'organisme, et doit donc être apporté par l'alimentation.

Il y a huit acides aminés essentiels : le tryptophane, la lysine, la méthionine, la phénylalanine, la thréonine, la valine, la leucine et l'isoleucine. Deux autres, l'histidine et l'arginine sont dits semi-essentiels car seuls les nourrissons ont besoin d'un apport exogène (on les trouve dans le lait maternel).

La phénylalanine est utilisée par les neurones pour synthétiser des neurotransmetteurs : dopamine, noradrénaline et adrénaline. Ces trois substances sont impliquées dans l'éveil, la vigilance, la motivation, et indirectement la minceur. La phénylalanine peut également entraîner la sécrétion de cholécystokinine, une substance qui donne au cerveau un signal de satiété. La méthionine est impliquée dans la biosynthèse des protéines, puisque toutes les chaînes protéiques démarrent par l'incorporation d'une méthionine en position N-terminale. Il est préférable de consommer la valine, la leucine et l'isoleucine regroupées. Les principales propriétés de ces trois acides aminés essentiels sont les suivantes : améliorer la coordination musculaire, réduire la perte musculaire observée chez les personnes âgées, normaliser le taux d'azote dans les muscles, augmenter l'énergie disponible, éliminer les manifestations de type hypoglycémique, augmenter l'endurance aux efforts physiques, maintenir les tendons et les cartilages en bonne santé, améliorer la pigmentation des globules rouges, régénérer et réparer les tissus musculaires, augmenter le métabolisme musculaire, éliminer une carence en isoleucine (troubles physiques, troubles mentaux). La thréonine est l'homologue hydroxylé de la valine. Ses principales propriétés sont les suivantes : assurer la croissance des cartilages, des ligaments, de l'émail, assurer l'équilibre protéique de l'organisme, améliorer le fonctionnement du coeur, du système nerveux central, de la musculature, de l'appareil digestif, de l'appareil intestinal, du système immunitaire. Le tryptophane est un acide aminé aromatique précurseur de l'hormone du sommeil, la mélatonine. Enfin, la lysine stimule la sécrétion d'hormone de croissance, un médiateur puissant de la construction musculaire, qui favorise aussi l'élimination des graisses de réserve.

C'est pourquoi un premier objet de l'invention est une poudre d'oeufs de cabillaud séchés comprenant :
- des acides aminés essentiels et,
- des lipides dont au moins :
   a. 50% en poids sont des phospholipides,
   b. 30% des acides gras totaux desdits lipides sont des acides gras oméga-3,
   c. 15% des acides gras totaux desdits lipides sont du DHA,
   d. 15% des acides gras totaux desdits lipides sont de l'EPA.

La mesure du pourcentage d'acides gras oméga-3, de DHA et d'EPA par rapport à la quantité d'acides gras totaux est réalisée par chromatographie en phase gazeuse selon la méthode NF EN ISO 5508 (juin 1995)

Avantageusement, environ 47% des acides gras totaux composant les lipides de la poudre de l'invention sont des acides gras oméga-3.

Avantageusement, environ 22% des acides gras totaux composant les lipides de la poudre de l'invention sont du DHA.

Avantageusement, environ 18% des acides gras totaux composant les lipides de la poudre de l'invention sont de l'EPA.

Préférentiellement, dans la poudre de l'invention, les acides gras oméga-3 représentent plus de 40% des acides gras totaux des phospholipides. De manière encore préférée, dans la poudre de l'invention, les acides gras oméga-3 représentent de 40 à 50% des acides gras totaux des phospholipides. De manière particulièrement préférée, dans la poudre de l'invention, les acides gras oméga-3 représentent environ 48% des acides gras totaux des phospholipides.

Préférentiellement, dans la poudre de l'invention, l'EPA représente plus de 15% des acides gras totaux des phospholipides et le DHA représente plus de 20% des acides gras totaux des phospholipides. De manière encore préférée, dans la poudre de l'invention, l'EPA représente de 15 à 20% des acides gras des phospholipides et le DHA représente de 20 à 30% des acides gras des phospholipides. De manière particulièrement préférée, dans la poudre de l'invention, l'EPA représente environ 19% des acides gras des phospholipides et le DHA représente environ 26% des acides gras des phospholipides.
Avantageusement, la poudre de l'invention comprend plus de 70% de protéines, de préférence plus de 75%.

Les acides aminés de la poudre de l'invention sont présents dans les protéines. Avantageusement, la poudre de l'invention comprend tous les acides aminés essentiels.

Avantageusement, chaque acide aminé essentiel représente plus de 1% en poids de la poudre de l'invention.

Avantageusement, la poudre de l'invention comprend plus de 2% en poids de phénylalanine, de manière particulièrement avantageuse plus de 3% en poids.

Avantageusement, la poudre de l'invention comprend plus de 5% en poids de leucine, de manière particulièrement avantageuse plus de 7% en poids.

Avantageusement, la poudre de l'invention comprend plus de 4% en poids de lysine, de manière particulièrement avantageuse plus de 5% en poids.

Avantageusement, la poudre de l'invention comprend plus de 3% en poids de isoleucine, de manière particulièrement avantageuse plus de 4% en poids.

Avantageusement, la poudre de l'invention comprend plus de 3% en poids de valine, de manière particulièrement avantageuse plus de 4% en poids.

Avantageusement, la poudre de l'invention comprend plus de 2% en poids de thréonine, de manière particulièrement avantageuse plus de 3% en poids.

Préférentiellement, la poudre de l'invention comprend également les 2 acides aminés semi-essentiels et indispensables aux nourrissons et aux enfants en période de croissance, l'histidine et l'arginine.

Préférentiellement, la poudre de l'invention comprend également des acides aminés non essentiels, de manière particulièrement préférée l'acide aspartique, la sérine, l'acide glutamique, la proline, l'alanine, la cystéine, la tyrosine et la glycine.

Un autre objet de l'invention concerne un procédé de préparation d'une poudre de l'invention comprenant les étapes suivantes :
(i) décongélation d'oeufs de cabillaud entiers congelés,
(ii) broyage des oeufs décongelés obtenus à l'étape (i)
(iii)séchage des oeufs broyés obtenus à l'étape (ii)
(iv)broyage du broyat sec obtenu à l'étape (iii).

Préférentiellement, l'étape (i) de décongélation a lieu à une température entre 0 et 4°C, de préférence d'environ 2°C, pendant 12 à 24 heures, de préférence 24 heures. Préférentiellement, l'étape (ii) de broyage a lieu avec un broyeur à couteaux type « grignoteuse ».

Préférentiellement, l'étape (iii) de séchage a lieu sur un plateau. Ainsi, la surface d'échange entre l'air chaud et le produit est maximale donc la durée du traitement plus courte.

Préférentiellement, l'étape (iii) de séchage a lieu en atmosphère azotée. L'absence d'air et donc d'oxygène limite l'oxydation du produit très riche en lipides et en particulier en oméga 3. Le vide est préférentiellement cassé avec de l'azote et non avec de l'air, là aussi car l'azote est un gaz neutre qui n'entraine pas l'oxydation du produit.

Selon un mode de réalisation, l'étape (iii) de séchage a lieu à environ 60°C.

Selon un autre mode de réalisation, l'étape (iii) de séchage a lieu à environ 90°C pendant un court laps de temps pour éliminer les micro-organismes puis à environ 60°C.

Préférentiellement, l'étape (iv) de broyage a lieu à très faible vitesse dans le broyeur pour limiter l'échauffement du produit et préserver ses qualités. Préférentiellement, l'étape (iv) de broyage a lieu également en atmosphère azotée.

Avantageusement, l'étape (iv) est suivie d'une étape (v) de micronisation. Cette étape de micronisation est réalisée dans le broyeur avec une grille très fine (100 microns).

La poudre obtenue à l'étape (iv) ou (v) peut être additionnée avec un antioxydant.

La poudre finale est conditionnée, par exemple dans des sacs plastiques, sous vide pour éviter toute perte de produit très fin donc volatile et tout contact avec de l'oxygène.

La granulométrie finale de la poudre est d'environ 100µm.

Cette granulométrie est avantageuse puisque la poudre de l'invention est destinée à être incorporée dans des compléments alimentaires.

Un autre objet de l'invention est un complément alimentaire comprenant une poudre de l'invention. Ce complément alimentaire se présente sous forme de gélule, capsule, comprimé, sirop, émulsion, suspension ou gel buvable.

Un autre objet de l'invention est l'utilisation d'une poudre de l'invention pour préparer un complément alimentaire. La poudre de l'invention est incorporée en même temps que les autres poudres dans la base liquide, les températures supérieures à 60°C étant à éviter sur de longues durées.

Un autre objet de l'invention est l'utilisation d'un complément alimentaire de l'invention chez la femme allaitante pour pallier ou prévenir ses carences en acides gras oméga-3.

Un autre objet de l'invention est un aliment destiné à l'alimentation animale ou aquacole comprenant une poudre selon l'invention.

Un autre objet de l'invention est l'utilisation d'une poudre de l'invention pour préparer un aliment destiné à l'alimentation animale ou aquacole.

Un autre objet de l'invention est une denrée alimentaire destinée à une alimentation particulière (DDAP) comprenant une poudre selon l'invention.

Les DDAP sont définies et réglementées par la Directive CE 1999/41/CE.

Les DDAP sont :
- des préparations pour nourrissons et préparations de suite,
- des denrées alimentaires à base de céréales et aliments pour bébés destinés aux nourrissons et aux enfants en bas âge,
- des aliments destinés à être utilisés dans les régimes hypocaloriques, destinés à la perte de poids,
- des aliments diététiques destinés à des fins médicales spéciales,
- des aliments adaptés à une dépense musculaire intense, surtout pour les sportifs, ou
- des aliments destinés à des personnes affectées d'un métabolisme glucidique perturbé.

En particulier, les DDAP de l'invention sont des aliments adaptés à une dépense musculaire intense, surtout pour les sportifs ou des aliments diététiques destinés à des fins médicales spéciales.

Un autre objet de l'invention est l'utilisation d'une poudre de l'invention pour préparer une DDAP.

Un autre objet de l'invention est l'utilisation d'un complément alimentaire, d'un aliment ou d'une denrée alimentaire destinée à une alimentation particulière selon l'invention pour stimuler les défenses immunitaires.

Un autre objet de l'invention est l'utilisation d'un complément alimentaire, d'un aliment ou d'une denrée alimentaire destinée à une alimentation particulière selon l'invention pour améliorer la gestion du poids.

Un autre objet de l'invention est l'utilisation d'un complément alimentaire, d'un aliment ou d'une denrée alimentaire destinée à une alimentation particulière selon l'invention pour améliorer la gestion du stress et/ou un ou plusieurs de ses symptômes.

Selon la présente invention, on entend par « stress » la sensation que l'on éprouve lorsque l'on est confronté à une situation à laquelle on ne croit pas pouvoir faire face correctement.

Le stress provoque un ensemble de réactions physiologiques et psychologiques qui épuise l'organisme si la situation se prolonge. Les symptômes du stress sont particulièrement: irritabilité, tension nerveuse, tension musculaire, palpitations, oppression thoracique, sensation de ventre noué, troubles du sommeil, fatigue, baisse du moral.

Un autre objet de l'invention est une poudre selon l'invention pour son utilisation en tant que médicament.

Un autre objet de l'invention est une poudre selon l'invention pour son utilisation dans la prévention ou le traitement de la dépression et/ou un ou plusieurs de ses symptômes.

Un autre objet de l'invention est l'utilisation d'une poudre selon l'invention pour la préparation d'un médicament destiné à prévenir ou traiter la dépression et/ou un ou plusieurs de ses symptômes.

On entend par « symptômes de la dépression » la tristesse, la fatigue, les insomnies, une perte d'appétit, des troubles de la concentration, de la mémoire, l'irritabilité, l'angoisse.

Les exemples suivants illustrent l'invention sans en limiter la portée.

### Exemple 1 : Flow chart du procédé de préparation d'une poudre de l'invention :

| |
|---|
| Oeufs de cabillaud congelés avec leur gangue |
| ↓ |
| Léger réchauffage à environ 2°C |
| ↓ |
| Broyage |
| ↓ |
| Disposition sur plateau de séchage |
| ↓ |
| Séchage doux sous vide, température de 90°C pendant un court laps de temps puis de 60°c |
| ↓ |
| Broyage sous azote |
| ↓ |
| Obtention d'une poudre fine (100 µm) |
| ↓ |
| Mélange avec un anti-oxydant (facultatif) |
| ↓ |
| Conditionnement sous vide |

### Exemple 2 : Analyse de la composition de la poudre obtenue par le procédé de l'exemple 1

### Analyse nutritionelle :

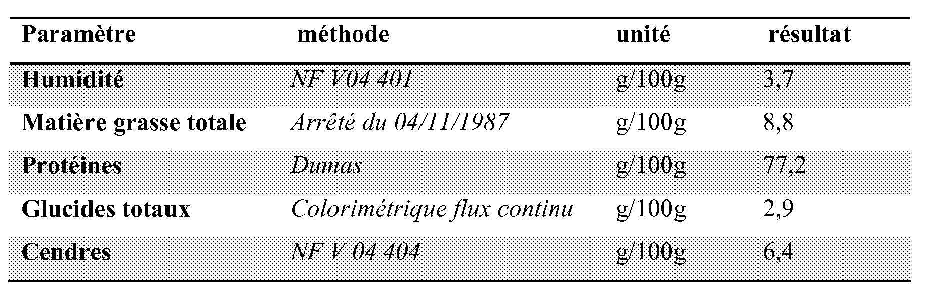

Kcalories/100g : 400
Kjoules/100g : 1687

### Composition de la partie lipidique par fractionnement des lipides sur colonne de silice après extraction de la matière grasse à froid par un mélange de solvants polaire/apolaire :

Les lipides sont composés à 33,04% de lipides neutres (triglycérides) et de 54,4% de lipides polaires (phospholipides). Le reste étant des composés lipidiques type pigments ou autres.

La teneur en DHA et EPA est très importante, en particulier dans la fraction des phospholipides.

**Tableau 1 : Répartition en % acides gras totaux :**

| | **EPA** | **DHA** | **Oméga 3 totaux** |
|---|---|---|---|
| **Lipides neutres** | 18,5 % | 14,4% | 38,7% |
| **Lipides polaires** | 18,9% | 26,30% | 48,9% |

La répartition des acides gras oméga 3 est mesurée par chromatographie en phase gazeuse des esters méthyliques des acides gras.

**Tableau 2 : Composition en acides aminés :**

| **Acides Aminés essentiels** | **En g/100g poudre** |
|---|---|
| **Phénylalanine** | 3,2 |
| **Leucine** | 7,1 |
| **Méthionine** | 1,8 |
| **Lysine** | 5,7 |
| **Isoleucine** | 4,2 |
| **Valine** | 4,6 |
| **Thréonine** | 3,5 |
| **Tryptophane** | 1,0 |

| **Acides Aminés semi-essentiels** | **En g/100g poudre** |
|---|---|
| **Histidine** | 1,7 |
| **Arginine** | 3,9 |
| | |

| **Acides Aminés (autres)** | **En g/100g poudre** |
|---|---|
| **Acide aspartique** | 6,2 |
| **Sérine** | 4,5 |
| **Acide glutamique** | 10,0 |
| **Proline** | 4,7 |
| **Alanine** | 5,5 |
| **Cystine** | 1,1 |
| **Tyrosine** | 3,2 |
| **Glycine** | 2,7 |

La composition en acides aminés est mesurée selon le règlement CE 152/2009 du 27/01/2009.

## Revendications

1. Poudre d'oeufs de cabillaud séchés comprenant
- des acides aminés essentiels et,
- des lipides dont au moins :
a. 50% en poids sont des phospholipides,
b. 30% des acides gras totaux desdits lipides sont des acides gras oméga-3,
c. 15% des acides gras totaux desdits lipides sont du DHA,
d. 15% des acides gras totaux desdits lipides sont de l'EPA.

2. Poudre selon la revendication 1, dont plus de 40% des acides gras totaux desdits phospholipides sont des acides gras oméga-3.

3. Poudre selon la revendication 1 ou 2, dont plus de 15% des acides gras totaux desdits phospholipides sont de l'EPA et plus de 20% des acides gras totaux desdits phospholipides sont du DHA.

4. Poudre selon l'une quelconque des revendications précédentes, comprenant plus de 70% de protéines.

5. Poudre selon l'une quelconque des revendications précédentes, comprenant tous les acides aminés essentiels et semi-essentiels.

6. Procédé de préparation d'une poudre selon l'une quelconque des revendications 1 à 5 comprenant les étapes suivantes :
(i) décongélation d'oeufs de cabillaud entiers congelés,
(ii) broyage des oeufs décongelés obtenus à l'étape (i)
(iii)séchage des oeufs broyés obtenus à l'étape (ii), préférentiellement à 90°C pendant un court laps de temps puis à 60°C
(iv)broyage du broyat sec obtenu à l'étape (iii), préférentiellement en atmosphère azotée,
(v) éventuellement micronisation du broyat obtenu à l'étape (iv).

7. Complément alimentaire comprenant une poudre selon l'une quelconque des revendications 1 à 5.

8. Aliment destiné à l'alimentation animale ou aquacole comprenant une poudre selon l'une quelconque des revendications 1 à 5.

9. Denrée alimentaire destinée à une alimentation particulière comprenant une poudre selon l'une quelconque des revendications 1 à 5.

10. Utilisation d'un complément alimentaire selon la revendication 7, d'un aliment selon la revendication 8 ou d'une denrée alimentaire destinée à une alimentation particulière selon la revendication 9 pour stimuler les défenses immunitaires.

11. Utilisation d'un complément alimentaire selon la revendication 7, d'un aliment selon la revendication 8 ou d'une denrée alimentaire destinée à une alimentation particulière selon la revendication 9 pour améliorer la gestion du poids.

12. Utilisation d'un complément alimentaire selon la revendication 7, d'un aliment selon la revendication 8 ou d'une denrée alimentaire destinée à une alimentation particulière selon la revendication 9 pour améliorer la gestion du stress et/ou un ou plusieurs de ses symptômes.

13. Poudre selon l'une quelconque des revendications 1 à 5 pour son utilisation en tant que médicament.

14. Poudre selon l'une quelconque des revendications 1 à 5 pour son utilisation dans la prévention ou le traitement de la dépression et/ou un ou plusieurs de ses symptômes.
